(19) **Europäisches Patentamt**
European Patent Office
Office européen des brevets

(11) **EP 0 806 670 B1**

(12) **EUROPÄISCHE PATENTSCHRIFT**

(45) Veröffentlichungstag und Bekanntmachung des Hinweises auf die Patenterteilung:
**22.04.2009 Patentblatt 2009/17**

(51) Int Cl.:
*G01N 33/68* (2006.01)       *G01N 33/86* (2006.01)

(21) Anmeldenummer: **97113801.1**

(22) Anmeldetag: **19.12.1990**

(54) **Verwendung eines Antikoagulant als Diagnostikum**

Use of an anticoagulant as a diagnostic agent

Utilisation d'un anticoagulant comme moyen de diagnostic

(84) Benannte Vertragsstaaten:
**AT BE CH DE DK ES FR GB GR IT LI LU NL SE**

(30) Priorität: **27.12.1989 DE 3942988**

(43) Veröffentlichungstag der Anmeldung:
**12.11.1997 Patentblatt 1997/46**

(62) Dokumentnummer(n) der früheren Anmeldung(en) nach Art. 76 EPÜ:
**91902118.8 / 0 509 026**

(73) Patentinhaber: **Boehringer Ingelheim International GmbH**
**55218 Ingelheim am Rhein (DE)**

(72) Erfinder: **Reutelingsperger, Christiaan**
**6212 GG Maastricht (NL)**

(56) Entgegenhaltungen:
**EP-A- 0 293 567**

- **The Journal of Immunology, Volume 143, No. 7, October 1989, Masato Umeda et al, "Effective production of monoclonal antibodies against phosphatidylerine: stereo-specific recognition of phosphatidylserine by monoclonal antibody", XP002900018**
- **The Journal of Biological Chemistry, Volume 264, No. 14, May 1989, Jonatan F. Tait et al, "Phospholipid Binding Properties of Human Placental Anticoagulant Protein-I, a Member of the Lipocortin Family", XP002900019**

**Beschreibung**

Gegenstand der vorliegenden Erfindung sind Mittel, insbesondere Annexine, die mit einer detektierbaren Substanz markiert sind.

Blut besteht aus speziellen ungebundenen Zellen, die in einem Plasma-Medium dispergiert sind. Der Zellinhalt ist von dem umgebenden Plasma durch sogenannte Plasma-Membranen getrennt. Diese Membranen sind aus Phospholipiden in Form einer Doppelschicht aufgebaut und aus assoziierten Proteinen, die teilweise diese Doppelschicht penetrieren bzw. aus ihnen herausragen.

Die verschiedenen Phospholipide sind nicht wahllos über die äußere und innere Hülle der Doppelschicht verteilt, sondern werden durch die Zelle in einer asymmetrischen Konfiguration gehalten (7,8). Während Phosphatidylcholin (PC) und Sphingomyelin (SPH) die dominierenden Spezies der äußeren Hülle sind, sind Phosphatidylserin (PS), Phosphatidylethanolamin (PE) und Phosphatidylinositol (PI) vorwiegend in der inneren Hülle lokalisiert, dem Cytosol zugewandt. Dieser energieverbrauchende Asymmetriezustand ist von außerordentlicher physiologischer Bedeutung. PC und SPH sind die am stärksten inerten Spezies der Phospholipidfamilie und weisen in krassem Gegensatz zu den anderen Speziesein äußerst neutrales Verhalten gegenüber den Plasmakomponenten auf. Diese Reaktionsträgheit der äußeren Hülle gegenüber den Plasmaproteinen ist absolute Voraussetzung, um den flüssigen Zustand des Blutes zu gewährleisten. Spezielle Plasmaproteine, die zur Blutgerinnungskaskade gehören, die sogenannten Koagulationsfaktoren, können nämlich den flüssigen Blutzustand in einen festen Zustand überführen, wenn sie aktiviert werden (9). Aktiviert werden können diese Koagulationsfaktoren durch Phospholipide wie Phosphatidylserin.

In manchen Fällen, z.B. nach Verletzung eines Blutgefäßes, ist es erforderlich, ja gar lebensnotwendig, daß die Koagulationsfaktoren aktiviert werden. In einer solchen Situation kann eine spezielle Blutzelle, das Plättchen, seine Membran-Asymmetrie durch Aktivierungsmechanismen, die Phosphatidylserin zur äußeren Hülle transportieren, aufgeben, wo es die Aktivierung der Koagulationsfaktoren unterstützt (10).

Diese planmäßige Änderung der Phospholipidzusammensetzung der äußeren Hülle der Plättchen Plasma-Membran, hat große physiologische Bedeutung für die Hämostase, wie beispielsweise durch das Scott-Syndrom (11) angedeutet.

Zur Physiologie gehört aber auch die Pathologie; so kann die Homöostase des Blutes in einigen Fällen auch in einen pathologischen Zustand abgleiten, wie es bei arteriellen, coronaren und venösen Thrombosen der Fall ist.

Diese hämostatischen Störungen sind meistens idiopathisch und machen es den Ärzten unmöglich, ihr Auftreten vorherzusagen und prophylaktische Therapien zu entwickeln.

Aufgabe der vorliegenden Erfindung war es, Hilfsmittel bereitzustellen, die hämostatische Störungen frühzeitig erkennen helfen.

Im Gegensatz zur Entwicklung der Symptome läuft die Entwicklung der Störungen in den meisten Fällen langsam ab. Während dieser Phase symptomloser Progression, dem sogenannten prothrombotischen Zustand, läuft lokal eine kontinuierliche Aktivierung des Koagulationssystems ab. Verbunden mit dieser lokalen Aktivierung ist in der Peripherie das Auftreten von Plättchen, die sich in einem frühen Zustand der Aktivierung befinden. Diese Plättchen haben bereits damit begonnen, die Phospholipidzusammensetzung ihrer äußeren Plasmamembranhülle zu ändern. Phosphatidylserin ist in der äußeren Hülle vorhanden. Hilfsmittel, die diesen sogenannten prothrombotischen Zustand diagnostizieren, wären von größter klinischer Bedeutung.

Gegenstand der vorliegenden Erfindung sind auch Hilfsmittel, die spezifisch Phosphatidylserin von Phosphatidylcholin unterscheiden können.

Neben dem Auftreten von schwach aktivierten Plättchen in der Peripherie, werden sich vollständig aktivierte Plättchen dort anheften, an der sich die Gefäßwand in pathologischer Kondition befindet. Diese Stelle ist als Auslöser für die Aktivierung des hämostatischen Systems anzusehen. Die Lokalisierung dieser pathologischen Stelle sowie die des sich hier bildenden Thrombus wäre von größtem therapeutischen Interesse.

Gegenstand der vorliegenden Erfindung sind daher auch Mittel mit denen der Ausgangspunkt der Aktivierung des hämostatischen Systems und/oder ein Thrombus zu lokalisieren ist.

Der Blutgerinnungsmechanismus stellt sich als eine Kaskade enzymatischer Reaktionen dar, an deren Ende die Bildung von Thrombin steht, das letztendlich Fibrinogen in Fibrin umwandelt. Verschiedene prokoagulante Reaktionen wie beispielsweise die Aktivierung von Prothrombin durch die Faktoren Xa und Va werden katalysiert durch Phospholipidoberflächen, an die die Koagulationsfaktoren binden.

Bei den Proteinen, die an Phospholipide binden und mit Phospholipidoberflächen abhängigen Prozessen interferieren, gibt es eine Familie, die in ihrer Bindung an Phospholipide $Ca^{2+}$ abhängig sind.

Zu dieser Familie, die auch Annexine genannt wird, gehört neben Lipocortin I, Calpactin I, Protein II, Lipocortin III, p67-Calelectrin auch das Vascular Antikoagulant Protein (VAC) und IBC, PAP, PAPI, PP4, Endonexin II und Lipocortin V.

Die gemeinsamen strukturellen Merkmale der Annexine sind wahrscheinlich die Grundlagen für ihre ähnlichen $Ca^{2+}$ und Phospholipidbindungseigenschaften. Obwohl diese generelle Eigenschaft für alle Annexine gilt, besteht eine klare Individualität hinsichtlich ihrer Affinität zu $Ca^{2+}$ und zu den verschiedenen Phospholipidarten.

Die physiologischen Funktionen der Annexine betreffen membranassoziierte Prozesse. Der grundlegende Mechanismus der gerinnungshemmenden Wirkung des VAC wurde als eine Hemmung der katalytischen Kapazität der Phospholipide durch die Bindung des VAC an ihre Oberfläche erkannt, wodurch die Bildung des gerinnungsfördernden Komplexes an ihrer Oberfläche verhindert wird.

Bindungsstudien haben gezeigt, daß sich VAC Calcium-abhängig mit prokoagulatorischen Phospholipiden reversibel assoziiert.

Auch andere bivalente Kationen aus der Reihe $Cd^{2+}$, $Zn^{2+}$, $Mn^{2+}$ und $Co^{2+}$ beeinflussen die Assoziation positiv, jedoch nicht in dem Maße wie $Ca^{2+}$.

Darüberhinaus wurde überraschenderweise gefunden, daß die VAC Adsorption an Phospholipide in Gegenwart von $Ca^{2+}$- und $Zn^{2+}$-Ionen außerordentlich positiv beeinflußt wird.

Überraschenderweise wurde festgestellt, daß VAC bei Plasma-Calziumkonzentrationen an Phosphatidylserin aber nicht an Phosphatidylcholin und Sphingomyelin bindet. VAC erkennt und bindet daher spezifisch periphere Plättchen mit PS in ihrer äußeren Membranhülle. Darüberhinaus erkennt VAC auch solche Stellen im vaskularen System spezifisch, die PS dem Blut aussetzen.

Diese Differenzierung bei den Phospholipiden macht VAC ebenso wie die anderen Annexine zu einem idealen Reagenz, um den sogenannten prothrombotischen Zustand, der sich wie oben geschildert darstellt, frühzeitig zu erkennen.

Durch die vorliegende Erfindung werden Mittel bereitgestellt, mit denen es überraschenderweise erstmals möglich ist, den prothrombotischen Zustand des vaskularen Systems zu erkennen. Ermöglicht wird diese Diagnose durch die Spezifität von Substanzen, von Mitteln, die in der Lage sind, den gegenüber dem Normalzustand veränderten, den prothrombotischen Zustand der Plättchen zu erkennen. Da sich dieser Zustand von dem Normalzustand der Plättchen dadurch unterscheidet, daß die äußere Hülle nur des prothrombotischen Plättchens Phosphatidylserin aufweist, ist zur Ausnutzung dieses erfindungsgemäßen Prinzips im Prinzip jedes Mittel geeignet, das Phosphatidylserin spezifisch von Phosphatidylcholin unterscheiden kann. Die erfindungsgemäß einsetzbaren Mittel sind durch ihre Spezifität zu Phosphatidylserin gekennzeichnet, die durch die in der Beschreibung angegebenen Bindungsversuche zu ermitteln ist.

Die Ausnutzung dieser Spezifität der erfindungsgemäßen Mittel macht es darüber hinaus auch möglich, den Ausgangspunkt für die Aktivierung des hämostatischen Systems und/oder den Thrombus zu lokalisieren.

Mit der vorliegenden Erfindung werden damit erstmals Hilfsmittel bereitgestellt, die es durch frühzeitige Diagnose eines möglicherweise sich entwickelnden gesundheitsgefährdenden Zustands ermöglichen, geeignete therapeutische Maßnahmen zu ergreifen.

Bevorzugte erfindungsgemäße Mittel sind anti-koagulierende Polypeptide, sofern sie die notwendige Spezifität zum Phospholipid Phosphatidylserin aufweisen.

Besonders bevorzugt ist die Familie der Annexine, insbesondere das VAC.

Um die erfindungsgemäßen Mittel, insbesondere VAC bzw. die anderen Annexine als Diagnostikum einsetzen zu können, werden sie in an sich bekannter Weise markiert. Als geeignete Marker bietet sich beispielsweise die Markierung mit fluoreszierenden Gruppen, oder die radioaktive Markierung an. Als vorteilhaft einsetzbarer Fluoreszenzmarker ist Fluoresceinisothiocyanat zu nennen, als vorteilhaft einsetzbare Radioaktivmarker die Radioisotope der Halogene, insbesondere die des Iod, beispielsweise $^{131}I$ oder $^{125}I$ oder auch Blei, Quecksilber, Thallium, Technetium oder Indium ($^{203}Pb$, $^{198}Hg$, $^{201}Tl$, $^{99m}Tc$, $^{111}In$) an.

Fluoresceinisothiocyanat (Serva) kann in an sich bekannter Weise (13) zur Markierung von VAC eingesetzt werden.

Auch möglich ist die Markierung mit einem paramagnetischen Kontrastagenz, das in einem MRI System (magnetic resonance imaging) detektierbar ist. Verwendet werden können Gadolinium, Kobalt, Nickel, Mangan oder Eisenkomplexe mit denen Konjugate als diagnostische Agenzien bereitgestellt werden können, die in einem MRI System detektierbar sind. In solchen Systemen wird ein starkes Magnetfeld verwendet, um die nuklearen Spinvektoren der Atome im Organismus auszurichten. Anschließend wird das Feld gestört, wodurch die Nuklei in ihren Ausgangszustand zurückkehren. Dieser Vorgang wird beobachtet und festgehalten.

Das so mit einem detektierbaren Marker versehene antikoagulierende Polypeptid wird dann auf intraarteriellem oder intravenösem Weg verabreicht. Die applizierte Menge ist so zu bemessen, daß sie für die nachfolgende Messung, nach genügender Inkubationszeit, ausreicht.

Zum Nachweis eines prothrombotischen Zustands wird dem zu untersuchenden Blut extrakorporal das erfindungsgemäß markierte Polypeptid oder Mittel zugegeben, gegebenenfalls in Anwesenheit eines weiteren, nicht die Plasma-Calciumkonzentration vermindernden Antikoagulants wie beispielsweise Heparin, woraufhin dann die mit spezifischen Zelltypen assoziierte Markierung analysiert wird.

Das erfindungsgemäß markierte Polypeptid kann sowohl in blutisotonischer wäßriger Lösung als auch mit Hilfsstoffen zum erfindungsgemäßen Zweck eingesetzt werden. Als Hilfsstoffe können beispielsweise TWEEN 80, Arginin, Phosphatpuffer und physiologisch verträgliche Konservierungsstoffe eingesetzt werden. Weitere Stoffe sind dem Fachmann bestens bekannt und können ebenfalls eingesetzt werden.

Zur Durchführung der radioaktiven Markierung verwendet man z.B. die bekannte Iodogen-Methode (12) oder die gebräuchliche Chloramin-T-Methode. Wegen seiner Halbwertszeit von 8 Tagen ist für die in vivo Diagnose $^{131}$I empfehlenswert. Das radioaktiv markierte Mittel wird in blutisotonischer wäßriger Lösung aufgenommen. Nach Sterilfiltration wird es injiziert. Die Ganzkörperszintigraphien werden mit einer Gammakamera z.B. für $^{131}$I 1, 2, 4 und 7 Tage nach der Injektion durchgeführt.

Neben den genuinen Formen der Annexine sind auch veränderte Formen für die erfindungsgemäße Verwendung einsetzbar.

Verwiesen sei insbesondere auf die in der EPA 0 293 567 beschriebenen Mutanten. Darüberhinaus sind auch die Fragmente oder chemisch modifizierte Derivate der Annexine verwendbar, die die Spezifität bezüglich der Phospholipide Phosphatidylserin/Phosphatidylcholin aufweisen und somit den prothrombotischen Zustand der beteiligten Plättchen erkennen können.

Gegenstand der vorliegenden Erfindung sind im Einzelnen:

- Antikoagulierendes Polypeptid aus der Familie der Annexine, vorzugsweise VAC, das mit einem detektierbaren Marker versehen ist.

- Antikoagulierendes Polypeptid, das als detektierbaren Marker die Fluoreszenzmarkierung vorzugsweise Fluoresceinisothiocyanat, ein Radioisotop eines Halogens, des Technetiums, Bleis, Quecksilbers, Thalliums oder des Indiums, besonders bevorzugt $^{131}$I oder $^{125}$I oder ein paramagnetisches Kontrastagenz enthält.

- Antikoagulierendes Polypeptid wie oben beschrieben zur Unterscheidung von Phophatidylserin und Phosphatidylcholin.

- Antikoagulierendes Polypeptid wie oben beschrieben zur Verwendung als Diagnostikum.

- Mittel, das neben einem mit einem detektierbaren Marker versehenem anti-koagulierendem Polypeptid aus der Familie der Annexine, vorzugsweise VAC zusätzlich einen Hilfsstoff beispielsweise physiologische Kochsalzlösung, TWEEN 80, Arginin und/oder Phosphatpuffer enthält, wobei gegebenenfalls ein nicht die Plasma-Calziumkonzentration reduzierendes Antikoagulant, vorzugsweise Heparin verwendet werden kann.

- Kit zum diagnostischen Nachweis des prothrombotischen Zustands oder des Ausgangspunktes der Aktivierung des hämostatischen Systems und/oder eines Thrombus, ein erfindungsgemäßes Mittel oder antikoagulierendes Polypeptid enthaltend, das in der Lage ist, Phosphatidylserin von Phosphatidylcholin zu unterscheiden.

Materialien und Methoden

VAC wurde entweder analog EPA 0 181 465 oder EPA 0 293 567 hergestellt. Die nachfolgenden Experimente wurden mit VACα durchgeführt, doch sind die Ergebnisse auch auf die anderen Annexine, insbesondere auch auf VACβ übertragbar.

Lipide

Dioleoyl-phosphatidylcholin (DOPC, Nr. P-1013)
Dioleoyl-phosphatidylethanolamin (DOPE, Nr. P-0510),
Cardiolipin (CL, Nr. C-5646), Dioleoylphosphatidylglycerol (DOPG, Nr. P-9664),
Phosphatidylinositol (PI, Nr. P-0639),
Dioleoyl-phosphatidsäure (DOPA, Nr. P-2767),
Stearylamin (SA, S-6755) und Eigelb Sphingomyelin (S-0756) wurden von der Firma Sigma Chemical Co bezogen.

Die Reinheit von DOPC und DOPE wurde durch Dünnschichtchromatographie überprüft. Dioleoylphosphatidylserin (DOPS) wurde durch Konversion von DOPC gemäß (1) hergestellt. $^{14}$C markiertes DOPS (spez. Akt. 100.000 dpm/μg) wurde von Amersham bezogen.

<u>Herstellung der Phospholipid-Doppelschichten auf Siliziumplatten.</u>

Phospholipid Doppelschichten wurden mit einem "Langmuir-film balance" (Lauda Typ FW-1) wie bei Corsel et al. (2) beschrieben aufgetragen. Hydrophile Siliziumplatten wurden 24 Stunden in 30 % Chromschwefelsäure und Wasser behandelt und in 50 % Ethanol/Wasser aufbewahrt. Vor ihrer Verwendung wurden sie gründlich mit einem Detergenz und Wasser gereinigt.

Der "film balance" wurde mit demineralisiertem Wasser und 50 $\mu$M CaCl$_2$ gefüllt. Auf diese Unterphase wurden 20 $\mu$l einer Lösung, die ca. 2g/l Phospholipid in Chloroform enthält, aufgebracht. Die DOPS Fraktionen in den Doppelschichten wurden mit $^{14}$C-markiertem DOPS in Mischung mit DOPC überprüft. Die aufgebauten Doppelschichten wurden von den Siliziumplatten mit dem Szintillations-Detergenz (Du Pont Formel-989) entfernt und die Totalradioaktivität wurde in einem Szintillationszähler gemessen.

<u>Messung der Bindung durch Ellipsometrie</u>

Die Adsorption von VAC an die Phospholipid-Doppelschichten wurde mit Hilfe eines automatischen Ellipsometers wie beschrieben gemessen (2,3).

Die Bindungsversuche wurden in einer hydrophilen Küvette, die 5 ml eines gerührten Puffers enthielt (0,05 M Tris/HCl; 0,1 M NaCl; pH=7,5; T=20°C) durchgeführt. Die divalenten Kationen wurden als Chloride schrittweise zugegeben.

Bei VAC-Konzentrationen <0,1 $\mu$g/ml wurde der Puffer, der die spezifische VAC Konzentration enthielt, kontinuierlich zugegeben, um für VAC eine ausreichende Pufferkapazität zu schaffen.

Aus den kombinierten Polarisier- und Analysier-Daten wurde der refraktive Index und die Dicke d des adsorbierten Films bestimmt (4). Die Menge $\Gamma$ der adsorbierten Proteinschicht wurde aus dem refraktiven Index und der Dicke mit Hilfe einer modifizierten Lorentz-Lorenz Gleichung [1] bestimmt (3,5):

$$[1] \quad \Gamma = 3d(n^2 - nb^2)/[(n^2+2)(r(nb^2+2)-v(nb^2-1))];$$

nb ist der refraktive Index des Puffers. Die Werte r=0,254 und v=0,71 wurden für die spezifische molare Refraktivität und des partiellen spezifischen Volumens eingesetzt (3).

<u>Ergebnisse</u>

<u>Der Effekt von divalenten Kationen auf die Bindung des VAC an Phospholipide.</u>

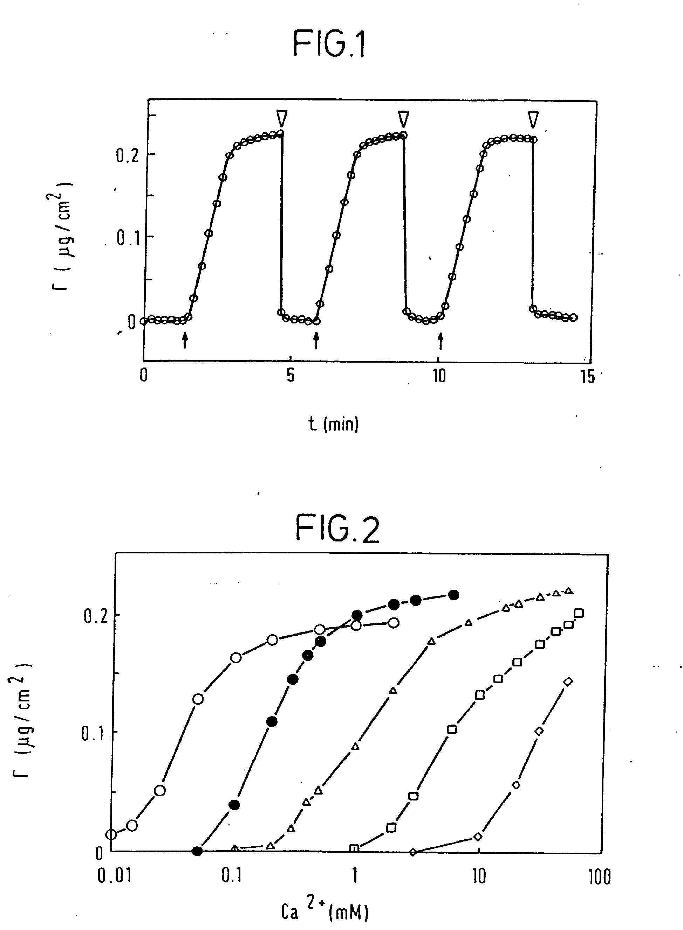

VAC bindet an Phospholipid-Membranen, die aus 20% DOPS/80% DOPC bestehen, in Abhängigkeit von der Calziumkonzentration. Anschließende Zugabe von EDTA führte zur sofortigen und vollständigen Desorption (Fig. 1). Durch die Veränderung der freien Ca$^{2+}$-Konzentration konnte die Adsorption mehrere Male wieder ausgelöst werden, ohne daß die adsorbierte Menge oder die Adsorptionsrate sich merklich veränderte. Irreversible Änderungen des VAC-Moleküls oder der Phospholipid-Doppelschichten durch die Adsorption oder Desorption sind daher nicht wahrscheinlich. Die Bindung war ebenfalls vollständig reversibel, wenn die Küvette mit Ca$^{2+}$-freiem Puffer gespült wurde.

Die Ca$^{2+}$-Abhängigkeit der VAC-Bindung an Phospholipide ist in Fig. 2 dargestellt. Die Ca$^{2+}$-Dosis-Wirkungs-Kurve zeigt ganz eindeutig eine Ca$^{2+}$-Konzentration, bei der die Hälfte der maximalen VAC-Adsorption erreicht wird: [Ca$^{2+}$]$_{1/2}$. Der [Ca$^{2+}$]$_{1/2}$-Wert hängt von der Zusammensetzung der Phospholipidoberfläche ab. Bei Phospholipidoberflächen, die 100%, 20%, 5% und 1% DOPS enthalten, wurden [Ca$^{2+}$]$_{1/2}$-Werte von 36 $\mu$M, 220 $\mu$M, 1,5 mM bzw. 8,6 mM gemessen (Tab. 1). Diese Ergebnisse stimmen recht gut überein mit dem [Ca$^{2+}$]$_{1/2}$-Wert von 53 $\mu$M, der für die Endonexin II (=VAC)- Bindung an äquimolaren Mischungen von PS/PC Vesikeln gemessen wurde (6). Die maximale Menge des adsorbierten Proteins ($\Gamma$max) war unabhängig von der DOPS Fraktion der Membran und betrug ca. 0,217 $\mu$g/cm$^2$. Keine Adsorption konnte bei reinen DOPC-Doppelschichten bis zu einer Ca$^{2+}$ Konzentration von 3mM festgestellt werden.

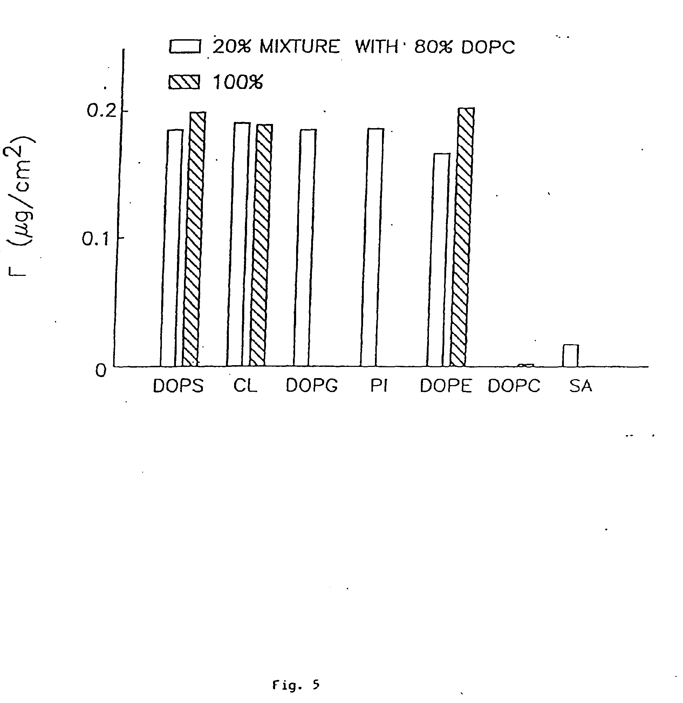

Die Adsorption von VAC an Phospholipid-Doppelschichten verschiedener Zusammensetzungen ist in Fig. 5 gezeigt. Auch hier wird deutlich, daß bei reinen DOPC-Doppelschichten praktisch keine Adsorption von VAC zu beobachten ist. Ebenfalls schwach ist die Adsorption des VAC an Stearylamid (SA).

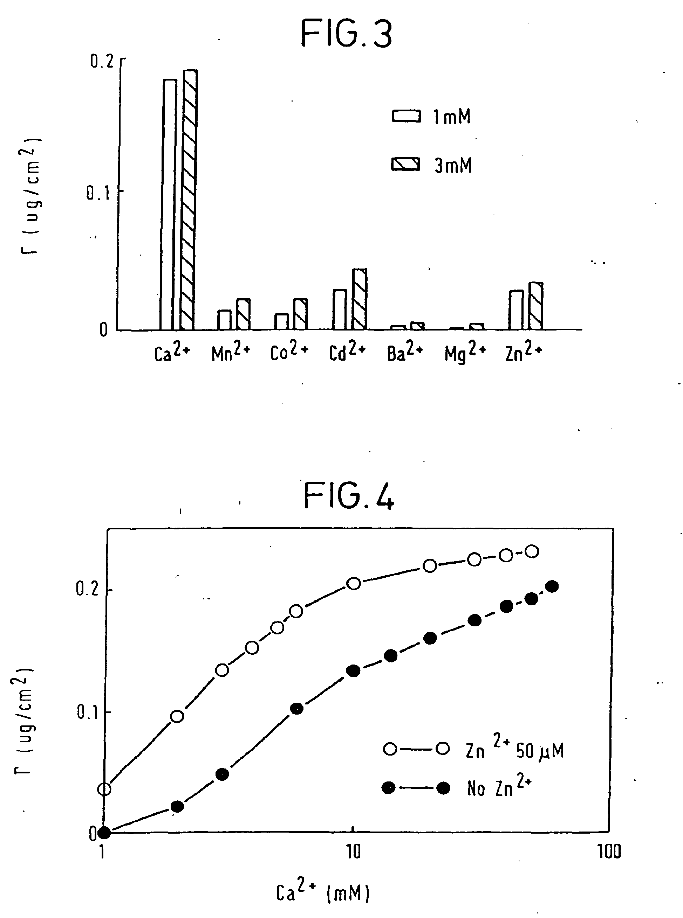

Bei Versuchen mit anderen Kationen als Ca$^{2+}$ wurde festgestellt, daß die Bindung von VAC an die Phospholipide in starkem Maße Ca$^{2+}$-spezifisch ist (Fig. 3). Cd$^{2+}$, Zn$^{2+}$, Mn$^{2+}$ und Co$^{2+}$ zeigten geringe Förderung der Bindung; Ba$^{2+}$ und Mg$^{2+}$ hatten keinen Einfluß. Diese Eigenschaft der Kationen kann in gewisser Weise mit ihren Ionenradien korreliert

werden.

<u>Zink Synergismus</u>

Hohe Konzentrationen an Zink-Ionen (1 mM) fördern nur in geringem Maß VAC-Adsorption (Fig. 3); 50 $\mu$M haben überhaupt keinen Einfluß auf die Adsorption. Überraschenderweise beeinflußt diese Konzentration die Bindung in Anwesenheit von $Ca^{2+}$; ein Synergismus ist zu beobachten. Der $[Ca^{2+}]_{1/2}$-Wert fiel von 8,6 auf 2,7 mM für Doppelschichten mit nur 1% DOPS ($[Zn^{2+}]$=50 $\mu$M.) (Fig. 4). 50 $\mu$M $[Zn^{2+}]$ liegt im normalen Bereich der Plasma Zink-Konzentrationen.

Diagnostische Verfahren

1. <u>in vitro</u> Diagnose

a) VAC wird nach an sich bekannten Verfahren mit der Fluoresceingruppe, beispielsweise mit Hilfe von Fluoreszeinisothiocyanat markiert (13); man erhält auf diese Weise VAC-FITC.

b) Blut eines Patienten wird in einem Plastikröhrchen gesammelt, das ein nicht die Plasma-Calziumkonzentration reduzierendes Antikoagulant (z.B. Heparin) und VAC-FITC enthält.

c) Nach der Durchmischung werden die Blutzellen unter Verwendung eines FACS (fluorescence activated cell sorter) analysiert. Diese Analyse bestimmt die Fluoreszenzintensität, die mit spezifischen Zelltypen assoziiert ist.

d) Die Analysenprofile zeigen die Menge an Plättchen mit gebundenem VAC-FITC, d.h. Plättchen mit exponiertem PS, und daher das Vorliegen eines prothrombotischen Zustands an. Dieser gesundheitsgefährdende Zustand kann auf diese Art frühzeitig erkannt und folglich frühzeitig therapiert werden.

2. <u>in vivo</u> Diagnose

a) VAC wird mit einem kurzlebigen Isotop, beispielsweise $^{131}$I nach an sich bekannten Verfahren markiert; man erhält $^{131}$I-VAC.

b) $^{131}$I-VAC wird intravenös einem Patienten appliziert.

c) Nach einer gewissen Inkubationszeit wird der Patient einer Ganz- oder Teilkörperscintigraphie ausgesetzt. Die Verteilung der Radioaktivität kann mit einer large-field-of-view Gamma-Kamera beobachtet werden.

d) Intravaskuläre Stellen mit $^{131}$I-VAC Akkumulation kennzeichnen die Stelle, an der die Thrombose fortschreitet. Geeignete, thromboseverhindernde bzw. thromboseheilende Maßnahmen können frühzeitig ergriffen werden.

1. <u>Radioaktive Markierung</u>

1.1 **Vorbereitungen**

1.1.1. <u>Herstellung eines 500 mmol/l Natriumphosphatpuffers</u>

24,5 g Natrium-di-hydrogenphosphatmonohydrat wurden in 1 l bidest. Wasser gelöst und zu einer Lösung von 35,5 g Di-natrium-hydrogenphosphat in 1 l bidest. Wasser bis zum pH 7,5 zugesetzt.

1.1.2. <u>Herstellung eines 20 mmol/L Natriumphosphatpuffers + 150 mmol NaCl (Elutionspuffer)</u>

2,76g Natrium-di-hydrogenphosphatmonohydrat wurden in 1 l bidest. Wasser gelöst und zu einer Lösung von 2,84 g Di-natrium-hydrogenphosphat in 1 l bidest. Wasser bis zum pH 7,2 zugesetzt. Durch Zugabe von 8,77 g NaCl (150 mmol) zu 1 l des Puffers wurde der Elutionspuffer hergestellt.

1.1.3. Equilibieren der Reinigungssäule

Eine PD-10-Säule (Sephadex G25, Fa. Pharmacia) wurde mit ca. 30 ml des Elutionspuffers equilibriert.

1.1.4. Vorbereitung des Reaktionsgefäßes

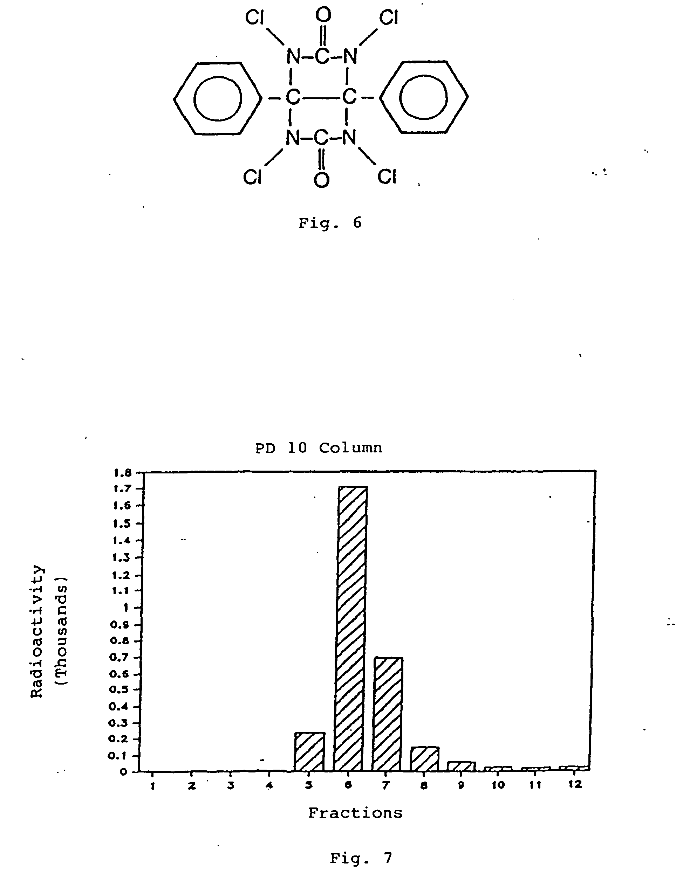

2 mg IODO-Gen (Molmasse: 432,09 g/mol; Fig. 6) wurden in 50 ml Dichlormethan, reinst, gelöst. 200 $\mu$l dieser Lösung wurden in ein 1,5 ml Eppendorf-Gefäß pipettiert und dann bei 37°C (Thermostatblock) das Lösungsmittel abgedampft. Im Reaktionsgefäß waren damit 8 $\mu$g ($1,85 \times 10^{-2}$ mmol) IODO-Gen feinst verteilt von der Wand aufgebracht.

1.1.5. Zur Markierung eingesetztes VAC-$\alpha$

Ausgegangen wurde von der Lösung von 50 mg VAC-$\alpha$ in 4 ml 20 mmol/l Natriumphosphatpuffer + 150 mmol/l NaCl, pH 7,2, verdünnt mit 1 ml bidest. Wasser. Molmasse VAC-$\alpha$: 34000 g/mol.

1.1.6. Zur Markierung eingesetztes J-125

Na$^{125}$J von der Fa. Dupont, NEN Products, mit 67,3 MBq (=1,82 mCi) Gesamtradioaktivität am Kalibrierungstag. Die spezifische Aktivität war 15,9 Ci/mg Jod = 1,98 kCi/mmol 1/2 $J_2$, das entsprach 0,115 $\mu$g Jod ($9,2 \times 10^{-7}$ mmol 1/2 $J_2$). Das aktive NaJ war in 5,5 $\mu$l 0,1 mol/l NaOH gelöst.

1.2. **Jodierung**

Alle Arbeiten wurden in Isotopenabzug hinter Bleiglasabschirmungen durchgeführt. 20 $\mu$l (= 200 $\mu$g) der unter 2.1.5 beschriebenen Lösung von VAC-$\alpha$ wurden in das IODO-Gen vorbehandelte Reaktionsgefäß überführt. Dieses wurde verschlossen und 20 Minuten bei Raumtemperatur geschüttelt. Danach wurde die Reaktionslösung mit einer Pipette auf die vorbereitete PD-10-Säule (s. 2.1.3.) aufgebracht. Das Reaktionsgefäß wurde noch mit 500 $\mu$l des Elutionspuffers (s. 2.1.2.) nachgespült und diese Lösung ebenfalls auf die PD-10-Säule gebracht. Das dabei abfließende Eluat wurde verworfen.

1.3. **Reinigung**

Durch Aufbringen von jeweils 0,5 ml Elutionspuffer (s. 2.1.2.) in 2-Minuten-Abständen, wurde nun das VAC-$\alpha$ [$^{125}$J] von dem noch freien $^{125}$J/Na $^{125}$J abgetrennt. Nach 12 Fraktionen war dieser Reinigungsschritt beendet. Der relative Aktivitätsgehalt der Fraktion wurde mit einem Labormonitor (GMZ) gemessen (Abb. 2).

Die Fraktionen 6 und 7 wurden vereinigt, mit Elutionspuffer auf genau 2,0 ml aufgefüllt, in 100 $\mu$l-Portionen aufgeteilt und bei -20°C eingefroren. Die Substanz stand in diesen Portionen für Analytik und Entwicklungsarbeiten zur Verfügung.

2. Analytischer Teil

2.1 **Gehaltsbestimmung**

Im chromogenen Substratassay wurde ein VAC-$\alpha$-Gehalt von 71,8 $\mu$g/2,0 ml Lösung gemessen.

2.2 **Radioaktivitätsbestimmung**

2.2.1. Gesamtradioaktivität

Nach dem Auftauen einer 100 $\mu$l-Portion wurden 50 $\mu$l dieser [$^{125}$] VAC-$\alpha$-Lösung zu 950 $\mu$l der inaktiven VAC-$\alpha$-Lösung (s. 2.1.5.) hinzupipettiert, gut vermischt und davon jeweils 50 $\mu$l zur Messung im LSC-Counter (Beckman) gebracht. 24,5 MBq (= 0,663 mCi) / 2,0 ml Gesamtlösung.

2.2.2. Spezifische Aktivität

Aus Gehaltsbestimmung und Gesamtradioaktivität ergab sich eine spezifische Aktivität von 341,5 MBq/mg = 11,61 TBq/mmol (9,23 mCi/mg = 313,8 Ci(mmol)

2.2.3. Bestimmung der proteingebundenen Radioaktivität durch TCA-Fällung

100 μl der VAC-α-Lösung wurden mit 50 μl 3% iger BSA-Lösung und 150 μl 40 %iger wäßriger Trichloressigsäure versetzt, gut durchgeschüttelt und 60 Minuten im Kühlschrank stehengelassen. Der entstandene Niederschlag wurde abzentrifugiert. Aliquote Mengen des Überstandes wurden im LSC-Counter zur Messung gebracht. Ergebnis: 99,3% der Radioaktivität waren fällbar.

2.2.4. Radioaktivitätsausbeute

Von den eingesetzten 67,3 MBq wurden 24,5 MBq im VAC-α wiedergefunden. Somit betrug die Aktivitätsausbeute 36,4 %.

2.3 **Modifikationsgrad**

Aus der spezifischen Aktivität und der eingesetzten Menge an inaktivem VAC-α wurde errechnet, daß statistisch jedes 6. VAC-α Molekül mit einem [125]J-Atom markiert wurde.

3.4 **Identität und Reinheit**

Unter Anwendung der SDS-PAGE (Gradientengel 7 - 17 %, nicht reduzierende Bedingungen) und der anschließenden Auswertung des Geles durch Silberfärbung (Oakley-Methode), Autoradiographie und Detektion unter dem Linearanalyzer (Fa. Berthold LB 282, Sonde LB 2820) (Abb. 3) wurde die Substanz im Vergleich zum eingesetzten VAC-α untersucht. Die Substanzen waren identisch, der Anteil an dimerem Produkt lag deutlich unter der für inaktive Chargen tolerierten Grenze von 8%.

Legende zu den Figuren

Fig. 1: Alternierende Adsorption und Desorption von VAC an eine Phospholipidoberfläche, induziert durch Erhöhung bzw. Erniedrigung der $Ca^{2+}$-Konzentration.
Die Adsorption von VAC (1 μg/ml) an eine 20 % DOPS/80 % DOPC Phospholipid-Doppelschicht. Zugabe, von $Ca^{2+}$ (3,4,6 mM) ist durch ↑ bzw. ∇ gekennzeichnet.

Fig. 2: Einfluß der Phospholipid-Zusammensetzung und der $Ca^{2+}$ Konzentration auf die Adsorption von VAC auf eine Phospholipidoberfläche.
o 100 % DOPS; o 20 % DOPS; △ 5 % DOPS; □ 1 % DOPS; 100 % DOPC; sämtliche Mischungen wurden ergänzt mit DOPC. [VAC] = 1 μg/ml.

Fig. 3: Effekt von bivalenten Ionen auf die Adsorption von VAC. VAC Adsorption an Doppelschichten aus 20 % DOPS und 80 % DOPC in Gegenwart der angegebenen Ionen (1 oder 3 mM). [VAC] = 1 μg/ml.

Fig. 4: Synergistischer Effekt von $Zn^{2+}$ auf die $Ca^{2+}$-abhängige Adsorption von VAC an die Phospholipidoberfläche. Der Effekt von $Ca^{2+}$ auf die VAC Adsorption an 1 % DOPS und 99 % DOPC in Gegenwart von 50 μM $Zn^{2+}$ wurde gemessen. [VAC] = 1 μg/ml.

Fig. 5: Adsorption von VAC an Phospholipid-Doppelschichten verschiedener Zusammensetzungen. VAC Adsorption an Dioleoylphosphatidylserin (DOPS), Cardiolipin (CL) und Dioleoylphosphatidylethanolamin (DOPE) entweder rein oder vermischt mit 80 % Dioleoylphosphatidylcholin (DOPC), an Dioleoylphosphatidylglycerol (DOPG), Phosphatidylinositol (PI) und Stearylamin vermischt mit 80 % DOPC oder an reines DOPC. [VAC] = 1 μg/ml; [$Ca^{2+}$] = 3 mM.

Fig. 6:
1,3,4,6-Tetrachloro-3α-6α-diphenyl-glycoluril (IODO-GEN)

Fig. 7: Radioaktivitätsverteilung in den Fraktionen 1-12.

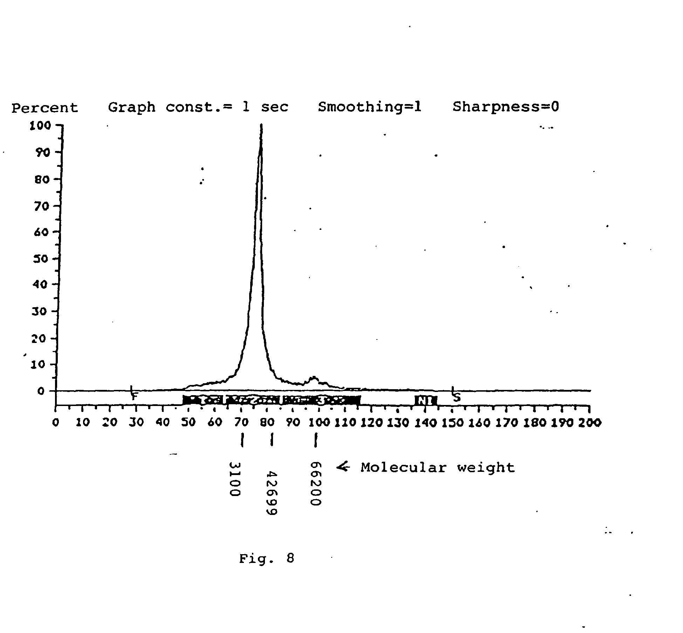

Fig. 8: Radioaktivitätsverteilung unter dem Linearanalyzer

Tabelle 1:

Evaluierung der Phospholipid-Doppelschichten, die auf Siliziumplatten aufgetragen waren.

| $^{14}$C-DOPS auf film balance % | Menge an Phospholipiden (ellipsometrisch) $\mu g/cm^2$ | Aktivität DPM | DOPS Fraktion gemessen % |
|---|---|---|---|
| 2 | 0,396 | 453 | 1,9 |
| 5 | 0,409 | 1133 | 4,5 |
| 20 | 0,401 | 5006 | 20 |
| 100 | 0,442 | 27174 | 99 |

Die kalkulierte Mischung wurde auf dem "film balance" aufgetragen. Die Menge der Doppelschicht wurde durch Ellipsometrie gemessen und die Aktivität des $^{14}$C-markierten DOPS wurde mit einem Beckmann 6S 3801 Szintillationszähler (s.d. <2%) gemessen und um die Hintergrundstrahlung korrigiert (60 DPM). Die DOPS-Fraktion in der Doppelschicht wurde durch Gleichung 2 berechnet:

$$\text{Fraktion} = \frac{\text{Menge}(\mu g/cm^2) \text{ x spez. Aktivität } (DPM.\mu g^{-1})}{\text{Aktivität } (DPM) \text{ x Fläche } (cm^2)}$$

Die spezifische Aktivität von DOPS betrug 100.000 $DPM.\mu g^{-1}$, die mit Phospholipiden besetzte Oberfläche 0,62 cm[2].

Tabelle 2:

Die Hälfte der maximalen VAC-Bindung an verschiedene Phospholipidoberflächen.

| Lipid (mol%/mol%) | $\Gamma max \pm S.D.$ ($\mu g/cm^2$) | $[Ca^{2+}]_{1/2} \pm S.D.$ mM |
|---|---|---|
| DOPS (100) | $0,195 \pm 0,025$ | $0,036 \pm 0,013$ |
| DOPS / DOPC (20/80) | $0,222 \pm 0,014$ | $0,22 \pm 0,06$ |
| DOPS / DOPC (5/95) | $0,229 \pm 0,004$ | $1,5 \pm 0,5$ |
| DOPS / DOPC (1/99) | $0,234 \pm 0,007$ | $8,6 \pm 2,5$ |
| Cardiolipin / DOPC (20/80) | $0,209 \pm 0,011$ | $0,039 \pm 0,022$ |
| DOPG / DOPC (20/80) | $0,212 \pm 0,003$ | $0,155 \pm 0,027$ |
| PI / DOPC (20/80) | $0,221 \pm 0,005$ | $0,47 \pm 0,05$ |
| DOPA / DOPC (20/80) | $0,207 \pm 0,006$ | $0,75 \pm 0,26$ |
| DOPE / DOPC (20/80) | $0,213 \pm 0,003$ | $0,86 \pm 0,21$ |
| Sphingomyelin / DOPC (20/80) | $0,225 \pm 0,014$ | $7 \pm 3$ |
| DOPC (100) | n.d. | >30 mM |

Die maximale VAC-Adsorption ($\Gamma max$) an die aufgeführten Phospholipidoberflächen zusammen mit der Calzium-Konzentration, die zur Hälfte der maximalen VAC-Bindung $[Ca^{2+}]_{1/2}$ führt, sind als Mittelwerte aus wenigstens drei verschiedenen Experimenten mit den entsprechenden Standard-Abweichungen angegeben.
n.d. = not determined = nicht bestimmt.

**Literaturverzeichnis**

1. Confurius, P & Zwaal, R. F. A. (1977) Biochim. Biophys. Acta 488, -42.

2. Corsel, J. W., Willems, G. M., Kop, J. M. M., Cuypers, P. A. & Hermens, W. Th. (1986) J. Colloid Interface Sci. 111, 544-554.

3. Cuypers, P. A., Corsel, J. W., Janssen, M. P., Kop, J. M. M., Hermens, W. TH. & Hemker, H. C. (1983) J. Biol. Chem. 258, 2426-2431.

4. McCrackin, F. L., Passaglia, E., Stromberg, R. R. & Steinberg, H. L. (1963) J.Res.Nat.Bur.Stand.Sect.A 67, 3-377.

5. Kop, J. M. M., Cuypers, P. A., Lindhout, Th., Hemker, H. C. & Hermens, W. Th. (1984) J. Biol. Chem. 259, 13993-13998.

6. Schlaepfer, D. D., Mehlman, T., Burgess, W. H. & Haigler. H. T. (1987) Proc. Natl. Acad. Sci. USA 84, 6078-6082.

7. Op den Kamp, J.A. F. Ann. Rev. Biochem. 1979, 48: 47-71.

8. Zwaal, R.F.A. Biochim. Biophys. Acta 1978, 515: 163-205.

9. Jackson, C.M. und Nemerson, Y. Ann. Rev. Biochem. 1980, 49: 765-811.

10. Bevers, E.M., Comfurius, P. und Zwaal, R.F.A. Biochim. Biophys. Acta 1983, 736: 57-66.

11. Rosing, J., Bevers, E.M., Comfurius, P., Hemker, H.C. can Dieijen, G., Weiss, H.J. und Zwaal, R.F.A. Blood 1985, 65: 1557-1561.

12. Fraker P.J., Speck, J.C., Biochem. Biophys. Res. Comm. 80, 849-857, 1978.

13. Reisher, J.I. & Orr, H.C., Anal. Biochem. 1968, 26, 178-179.

## Patentansprüche

1. Kit zum diagnostischen Nachweis des prothrombischen Zustands oder des Ausgangspunktes der Aktivierung des hämostatischen Systems und/oder eines Thrombus, enthaltend ein antikoagulierendes Peptid, das in der Lage ist, Phosphatidylserin von Phosphatidylcholin zu unterscheiden, wobei das antikoagulierende Peptid ausgewählt ist aus der Familie der Annexine und mit einem detektierbaren Marker versehen ist.

2. Kit gemäß Anspruch 1, bei dem es sich bei dem Annexin um VAC handelt.

3. Kit gemäß einem der Ansprüche 1 und 2, bei dem der detektierbare Marker ein Fluoresceinisothiocyanat, ein Radioisotop eines Halogens, des Technetiums, Bleis, Quecksilbers, Thalliums oder des Indiums oder ein paramagnetisches Kontrastagenz ist.

4. Kit gemäß einem der Ansprüche 1 bis 3, wobei der Kit zusätzlich ein nicht die Plasma-Calciumkonzentration reduzierendes Antikoagulant enthält.

5. Kit gemäß Anspruch 4, bei dem es sich bei dem nicht die Plasma-Calciumkonzentration reduzierenden Antikoagulant um Heparin handelt.

6. Antikoagulierendes Polypeptid aus der Familie der Annexine, wobei das Annexin mit einem detektierbaren Marker versehen ist, zur Verwendung als Mittel zur Diagnose des prothrombischen Zustands oder des Ausgangspunkts der Störung oder Aktivierung des hämostatischen Systems und/oder eines Thrombus.

7. Antikoagulierendes Polypeptid gemäß Anspruch 6, wobei es sich bei dem Annexin um VAC handelt.

8. Antikoagulierendes Polypeptid gemäß einem der Ansprüche 6 und 7, bei dem der detektierbare Marker ein Fluoresceinisothiocyanat, ein Radioisotop eines Halogens, des Technetiums, Bleis, Quecksilbers, Thalliums oder des Indiums oder ein paramagnetisches Kontrastagenz ist.

## Claims

1. Kit for the diagnostic detection of the prothrombotic state or the starting point of the activation of the haemostatic system and/or of a thrombus, containing an anticoagulant polypeptide which is capable of distinguishing between phosphatidyl choline and phosphatidyl serine, the anticoagulant polypeptide being selected from the family of annexins and being provided with a detectable marker.

2. Kit according to claim 1, wherein the annexin is VAC.

3. Kit according to one of claims 1 and 2, wherein the detectable marker is a fluorescein isothiocyanate, a radioisotope of a halogen, technetium, lead, mercury, thallium or indium, or a paramagnetic contrast agent.

4. Kit according to one of claims 1 to 3, wherein the kit additionally contains an anticoagulant which does not reduce the plasma calcium concentration.

5. Kit according to claim 4, wherein the anticoagulant which does not reduce the plasma calcium concentration is heparin.

6. Anticoagulant polypeptide selected from the family of annexins, the annexin being provided with a detectable marker, for use as a means of diagnosing the prothrombotic state or the starting point of the malfunction or activation of the haemostatic system and/or of a thrombus.

7. Anticoagulant polypeptide according to claim 6, wherein the annexin is VAC.

8. Anticoagulant polypeptide according to one of claims 6 and 7, wherein the detectable marker is a fluorescein isothiocyanate, a radioisotope of a halogen, technetium, lead, mercury, thallium or indium, or a paramagnetic contrast agent.

## Revendications

1. Kit pour la mise en évidence diagnostique de l'état prothrombique ou du point de départ de l'activation du système hémostatique et/ou d'un thrombus contenant un peptide anticoagulant qui est en mesure de distinguer la phosphatidylsérine de la phosphatidylcholine, où le peptide anticoagulant est choisi dans la famille des annexines et est muni d'un marqueur détectable.

2. Kit selon la revendication 1 où l'annexine est VAC.

3. Kit selon l'une des revendications 1 et 2 où le marqueur détectable est un isothiocyanate de fluorescéine, un radioisotope d'un halogène, du technétium, du plomb, du mercure, du thallium ou de l'indium ou un agent de contraste paramagnétique.

4. Kit selon l'une des revendications 1 à 3 où le kit contient en outre un anticoagulant ne réduisant pas la concentration plasmatique de calcium.

5. Kit selon la revendication 4 où l'anticoagulant ne réduisant pas la concentration plasmatique de calcium est l'héparine.

6. Polypeptide anticoagulant de la famille des annexines où l'annexine est munie d'un marqueur détectable, destiné à être utilisé comme moyen pour le diagnostic de l'état prothrombique ou du point de départ de la perturbation ou de l'activation du système hémostatique et/ou d'un thrombus.

7. Polypeptide anticoagulant selon la revendication 6 où l'annexine est VAC.

8. Polypeptide anticoagulant selon l'une des revendications 6 et 7 où le marqueur détectable est un isothiocyanate de fluorescéine, un radioisotope d'un halogène, du technétium, du plomb, du mercure, du thallium ou de l'indium ou un agent de contraste paramagnétique.

# FIG.1

# FIG.2

## FIG.3

## FIG.4

Fig. 5

14

Fig. 6

PD 10 Column

Fig. 7

Fig. 8

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur

- **CONFURIUS, P ; ZWAAL, R. F. A.** *Biochim. Biophys. Acta,* 1977, vol. 488, 42
- **CORSEL, J. W. ; WILLEMS, G. M. ; KOP, J. M. M. ; CUYPERS, P. A. ; HERMENS, W. TH.** *J. Colloid Interface Sci.,* 1986, vol. 111, 544-554
- **CUYPERS, P. A. ; CORSEL, J. W. ; JANSSEN, M. P. ; KOP, J. M. M. ; HERMENS, W. TH. ; HEMKER, H. C.** *J. Biol. Chem.,* 1983, vol. 258, 2426-2431
- **MCCRACKIN, F. L. ; PASSAGLIA, E. ; STROMBERG, R. R. ; STEINBERG, H. L.** *J.Res.Nat.Bur.Stand.Sect.A,* 1963, vol. 67, 3-377
- **KOP, J. M. M. ; CUYPERS, P. A. ; LINDHOUT, TH. ; HEMKER, H. C. ; HERMENS, W. TH.** *J. Biol. Chem.,* 1984, vol. 259, 13993-13998
- **SCHLAEPFER, D. D. ; MEHLMAN, T. ; BURGESS, W. H. ; HAIGLER. H. T.** *Proc. Natl. Acad. Sci. USA,* 1987, vol. 84, 6078-6082

- **OP DEN KAMP, J.A. F.** *Ann. Rev. Biochem.,* 1979, vol. 48, 47-71
- **ZWAAL, R.F.A.** *Biochim. Biophys. Acta,* 1978, vol. 515, 163-205
- **JACKSON, C.M. ; NEMERSON, Y.** *Ann. Rev. Biochem.,* 1980, vol. 49, 765-811
- **BEVERS, E.M. ; COMFURIUS, P. ; ZWAAL, R.F.A.** *Biochim. Biophys. Acta,* 1983, vol. 736, 57-66
- **ROSING, J. ; BEVERS, E.M. ; COMFURIUS, P. ; HEMKER, H.C. ; CAN DIEIJEN, G. ; WEISS, H.J. ; ZWAAL, R.F.A.** *Blood,* 1985, vol. 65, 1557-1561
- **FRAKER P.J. ; SPECK, J.C.** *Biochem. Biophys. Res. Comm.,* 1978, vol. 80, 849-857
- **REISHER, J.I. ; ORR, H.C.** *Anal. Biochem.,* 1968, vol. 26, 178-179